# EUROPEAN PATENT APPLICATION

(11) **EP 3 859 323 A1**
(43) Date of publication of application: **04.08.2021**
(21) Application number: 19867756.9
(22) Date of filing: 02.09.2019
(51) Int. Cl.: G01N 27/416, G01N 27/48

(54) **METHOD, DEVICE, AND PROGRAM FOR DETECTING DEGREE OF HYBRIDIZATION OF NUCLEIC ACID**

(30) Priority: 28.09.2018 JP 2018183439
(71) Applicant: YOKOWO CO., LTD., Kita-ku Tokyo 114-8515 (JP)
(72) Inventor: TAKASE Shintaro, Tokyo 114-8515 (JP); MIYAGAWA Kouta, Tokyo 114-8515 (JP); USAMI Eiji, Tokyo 114-8515 (JP)
(74) Representative: Sibley, Lara Ann
(86) International application number: PCT/JP2019/034380
(87) International publication number: WO 2020/066474

(57) **Abstract**

This device (10) includes a calculator (100). The calculator (100) is for calculating a potential difference (ΔE) between a first potential (E1) of a first oxidation wave (O1) and a second potential (E2) of a second oxidation wave (O2). In the first oxidation wave (O1), the first potential (E1) has a first current value (I1) in a range less than a potential (Ep⁰) at a peak current value (I⁰) of the first oxidation wave (O1). In the second oxidation wave (O2), the second potential (E2) has the first current value (I1) in a range less than a potential (Ep^{0'}) at a peak current value (I^{0'}) of the second oxidation wave (O2).

## Description

### FIELD

The present invention relates to a method, a device and a program for detecting a degree of nucleic acid hybridization.

### BACKGROUND

Hybridization is often used to detect nucleic acids (such as deoxyribonucleic acid (DNA) or ribonucleic acid (RNA)) that have specific sequences. Nucleic acids hybridize to probes that have sequences complementary to sequences of the nucleic acids. The degree of nucleic acid hybridization can be quantitatively analyzed using an electrochemical approach.

NPL 1 describes an example of a method for quantitatively analyzing a degree of nucleic acid hybridization using an electrochemical approach. In this case, a sample containing microRNA (miRNA) is used in cyclic voltammetry (CV) to obtain a pre-hybridization voltammogram (oxidation wave and reduction wave) and a post-hybridization voltammogram (oxidation wave and reduction wave). When the peak current value I⁰ for the pre-hybridization voltammogram (a pre-hybridization oxidation wave voltammogram has a peak current value I⁰ at potential Ep⁰) and the current value I for the potential Ep⁰ of the post-hybridization oxidation wave voltammogram are measured for identical work electrodes, the current value I is smaller than the peak current value I⁰. Therefore, the ratio I/I⁰ of the current value I with respect to the peak current value I⁰ can function as an indicator for quantitative analysis of a degree of nucleic acid hybridization.

### [CITATION LIST]

### [PATENT LITERATURE]

[NPL 1] Gene sensors based on peptide nucleic acid (PNA) probes: Relationship between sensor sensitivity and probe/target duplex stability. Analyst, 2005, 130, 1478-82.

### SUMMARY

### [TECHNICAL PROBLEM]

The present inventors have investigated methods of quantitatively analyzing a degree of nucleic acid hybridization using a novel indicator which is different from the indicator described in NPL 1 (the relationship between the peak current value I⁰ and current value I). When the peak current value I⁰ and current value I are essentially equal (such as when the amounts of nucleic acids in each sample are very small or when virtually no hybridization has taken place), for example, it is difficult to accurately calculate the relationship (for example, the ratio or difference) between the peak current value I⁰ and current value I, and it is difficult to use the relationship between the peak current value I⁰ and current value I for high-precision quantitative analysis of a degree of nucleic acid hybridization. Also, when it is difficult to accurately measure the peak current value I⁰ (such as when the voltammogram near the peak current value I⁰ is not steep, thus resulting in a wide range of potentials in which the peak current value I⁰ and current values near the peak current value I⁰ are obtained, and making it difficult to establish a unique potential Ep⁰), it becomes difficult to accurately calculate the relationship (for example, the ratio or difference) between the peak current value I⁰ and current value I, and it is difficult to use the relationship between the peak current value I⁰ and current value I for high-precision quantitative analysis of a degree of nucleic acid hybridization.

One object of the invention is to obtain a novel indicator for quantitative analysis of a degree of nucleic acid hybridization. Other objects of the invention will become apparent from the description throughout the present specification.

### [SOLUTION TO PROBLEM]

One aspect of the invention is a method for detecting a degree of nucleic acid hybridization using a first oxidation wave and a second oxidation wave, wherein:
the method includes calculating the potential difference between a first potential at which, in the first oxidation wave, a first current value is obtained in a range below the potential for the peak current value of the first oxidation wave, and a second potential at which, in the second oxidation wave, a first current value is obtained in a range below the potential for the peak current value of the second oxidation wave.

Another aspect of the invention is a device for detecting a degree of nucleic acid hybridization using a first oxidation wave and a second oxidation wave, wherein:
the device comprises a calculator which calculates the potential difference between a first potential at which, in the first oxidation wave, a first current value is obtained in a range below the potential for the peak current value of the first oxidation wave, and a second potential at which, in the second oxidation wave, a first current value is obtained in a range below the potential for the peak current value of the second oxidation wave.

Yet another aspect of the invention is a program that causes a computer to function as a device for detecting a degree of nucleic acid hybridization using a first oxidation wave and a second oxidation wave, wherein:
the program causes the computer to:
calculate the potential difference between a first potential at which, in the first oxidation wave, a first current value is obtained in a range below the potential for the peak current value of the first oxidation wave, and a second potential at which, in the second oxidation wave, a first current value is obtained in a range below the potential for the peak current value of the second oxidation wave.

### [ADVANTAGEOUS EFFECTS OF INVENTION]

According to the aforementioned aspects of the invention it is possible to obtain a novel indicator for quantitative analysis of a degree of nucleic acid hybridization.

### BRIEF DESCRIPTION OF DRAWINGS

Fig. 1 is a diagram illustrating a device according to embodiment 1.
Fig. 2 is a diagram illustrating a device according to embodiment 2.
Fig. 3 is a diagram illustrating an example of reference data stored in the memory shown in Fig. 2.
Fig. 4 is a diagram showing the hardware configuration of a device.
Fig. 5 is a diagram showing a first modified example of Fig. 1.
Fig. 6 is a diagram showing a second modified example of Fig. 1.
Fig. 7 is a diagram showing a third modified example of Fig. 1.
Fig. 8 is a diagram showing the correlation between miRNA hybridization analysis using the current value ratio I/I⁰ and miRNA hybridization analysis using the potential difference ΔE.

### DESCRIPTION OF EMBODIMENTS

Embodiments of the invention will now be described with reference to the accompanying drawings. In all of the diagrams, corresponding constituent elements are indicated using like reference numerals and will not be redundantly explained.

In the following explanation, the calculator 100, determiner 110 and memory 120 are shown not as hardware unit configurations but rather as functional unit blocks. The calculator 100, determiner 110 and memory 120 are provided by an optional combination of hardware and software, which may essentially be any type of computer CPU, a memory, a program that forms the constituent elements in the diagram that are loaded into the memory, a storage medium such as a hard disk that stores the program, and a network connection interface. A variety of different modified examples for the method and device are possible.

### (Embodiment 1)

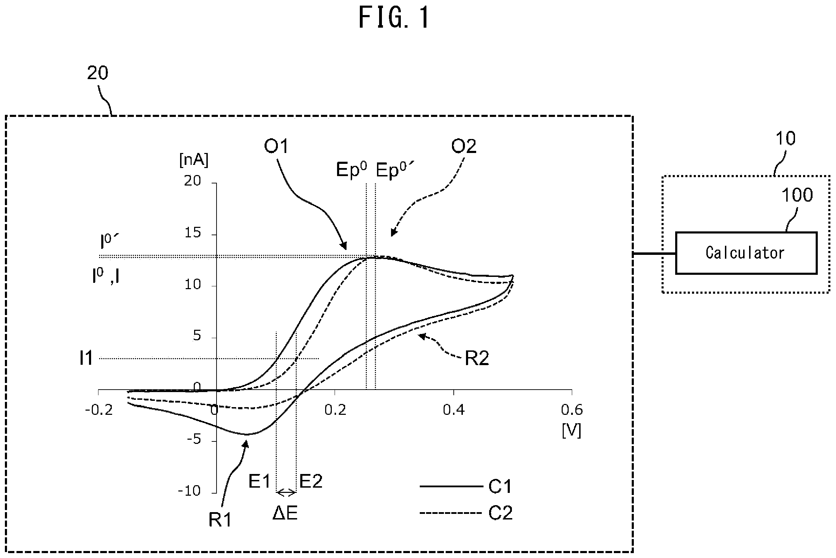

Fig. 1 is a diagram illustrating a device 10 according to embodiment 1. In the graph in the measurement system 20 of Fig. 1, the ordinate represents the current (nA) for the voltammogram and the abscissa represents potential (V) for the voltammogram.

Fig. 1 will now be used for general explanation of the device 10. The device 10 uses a first oxidation wave O1 and a second oxidation wave O2 to detect the degree of nucleic acid hybridization. The device 10 includes a calculator 100. The calculator 100 calculates the potential difference ΔE between the first potential E1 of the first oxidation wave O1 and the second potential E2 of the second oxidation wave O2. In the first oxidation wave O1, the first potential E1 has a first current value I1 in a range of less than the potential Ep⁰ at the peak current value I⁰ of the first oxidation wave O1. In the second oxidation wave O2, the second potential E2 has a first current value I1 in a range of less than the potential Ep^{0'} at the peak current value I^{0'} of the second oxidation wave O2.

With this configuration it is possible to obtain a novel indicator for quantitative analysis of the degree of nucleic acid hybridization. Specifically, this configuration allows the potential difference ΔE to be calculated by the calculator 100. As explained below, the potential difference ΔE can serve as an indicator for quantitative analysis of the degree of nucleic acid hybridization.

Moreover, this configuration does not require calculation of the relationship (for example, the ratio or difference) between the peak current value I⁰ and current value I, even when the peak current value I⁰ of the first oxidation wave O1 and the current value I of the second oxidation wave O2 (the second oxidation wave O2 has a second current value (current value I) at the potential Ep⁰ for the peak current value I⁰ of the first oxidation wave O1), are essentially equal. Thus, even when the peak current value I⁰ of the first oxidation wave O1 and the current value I of the second oxidation wave O2 are essentially equal, it is possible to quantitatively analyze the degree of nucleic acid hybridization to a high degree of precision.

According to one example, the potential difference ΔE can be calculated even when the peak current value I⁰ of the first oxidation wave O1 and the current value I of the second oxidation wave O2 are essentially equal, such as when the current value I of the second oxidation wave O2 is 90% to 110% of the peak current value I⁰ of the first oxidation wave O1.

In addition, with the aforementioned configuration it is not necessary to calculate the relationship (for example, the ratio or difference) between the peak current value I⁰ and current value I, even when it is difficult to accurately measure the peak current value I⁰ of the first oxidation wave O1 (such as when the first oxidation wave O1 near the peak current value I⁰ is not steep, thus resulting in a wide range of potentials in which the peak current value I⁰ and current values near the peak current value I⁰ are obtained, and making it difficult to establish a unique potential Ep⁰). Thus, even when it is difficult to accurately measure the peak current value I⁰, it is still possible to quantitatively analyze the degree of nucleic acid hybridization to a high degree of precision.

Fig. 1 will now be used for a more detailed explanation of the device 10.

In the example shown in Fig. 1, the measurement system 20 measures a voltammogram C1 and a voltammogram C2 by cyclic voltammetry (CV). The voltammogram C1 includes the first oxidation wave O1 and a first reduction wave R1. The voltammogram C2 includes the second oxidation wave O2 and a second reduction wave R2.

According to another example, the measurement system 20 may measure each voltammogram by a method other than CV, such as differential pulse voltammetry (DPV). In this example as well, each voltammogram includes an oxidation wave. The potential difference ΔE can therefore be calculated by the same method as explained using Fig. 1.

The measurement system 20 has an electrode (work electrode) for measurement of the voltammogram C1 and voltammogram C2. The measurement system 20 may also have multiple electrodes (multiple work electrodes). In this case the calculator 100 will be able to calculate multiple potential differences ΔE respectively for the multiple electrodes. The calculator 100 may statistically process the multiple potential differences ΔE, by calculating the median or average for the multiple potential differences ΔE, for example.

In the example shown in Fig. 1, the first oxidation wave O1 represents the measurement results for a sample before hybridization while the second oxidation wave O2 represents the measurement results for the sample after the hybridization. The sample before hybridization may include a probe immobilized on the electrode (work electrode) of the measurement system (the sample before hybridization may or may not include nucleic acids), and the sample after the hybridization may include nucleic acids hybridized to the probe.

In the example shown in Fig. 1, the first oxidation wave O1 and the second oxidation wave O2 represent measurement results for microRNA (miRNA). The miRNA may be extracted from blood. It is generally difficult to obtain a sample which contains a large amount of miRNA from blood. Therefore, changes in the miRNA voltammogram (such as oxidation wave) due to hybridization can be extremely small. It may therefore be difficult to quantitatively analyze a degree of miRNA hybridization using the relationship (for example, the ratio or difference) between the peak current value I⁰ and current value I. If the example shown in Fig. 1 is used, however, a clear potential difference ΔE can be produced even in the case of miRNA hybridization. It is thus possible to quantitative analyze the degree of miRNA hybridization to a high degree of precision.

According to another example, each oxidation wave may represent measurement results for other type of nucleic acid than miRNA. For example, it may represent measurement results for DNA or it may represent measurement results for other RNA than miRNA.

The user of the device 10 may appropriately determine the first current value I1 depending on different conditions (such as the first oxidation wave O1 and the second oxidation wave O2).

According to one example, the first current value I1 may be selected from a range of current values in which variation (such as standard deviation) in the potential difference ΔE is kept to within a fixed range, and for example, it may be 10% to 90% of the peak current value I⁰ of the first oxidation wave O1 (a potential difference ΔE of within 10% to 90% of the peak current value I⁰ of the first oxidation wave O1, for example, is kept to within a fixed range from the mean value for the potential difference ΔE at 10% to 90% of the peak current value I⁰ of the first oxidation wave O1, at any current value). The calculator 100 may also calculate the potential difference ΔE for a plurality of current value ratios I1/I⁰ (for example, current value ratios I1/I⁰ of 0.20, 0.30 and 0.40). The calculator 100 may statistically process the multiple potential differences ΔE, such as calculating the median or average for the multiple potential differences ΔE.

The reason for the potential difference ΔE produced in Fig. 1 will now be explained.

The potential of an electrode (work electrode) in a measurement system 20 can fall due to a negative overall charge ΔQ produced by the hybridized target nucleic acid. During measurement of the oxidation wave, an electrical double layer of capacitance C is formed in the work electrode. The reduction in potential of the work electrode can be estimated as ΔQ/C. Therefore, the post-hybridization oxidation wave (the second oxidation wave O2 in the example shown in Fig. 1) can shift toward a higher potential by ΔQ/C from the pre-hybridization oxidation wave (the first oxidation wave O1 in the example shown in Fig. 1). The potential difference ΔE allows estimation of the amount of shift from the pre-hybridization oxidation wave (the first oxidation wave O1 in the example shown in Fig. 1) to the post-hybridization oxidation wave (the second oxidation wave O2 in the example shown in Fig. 1), which is approximately equal to ΔQ/C. The potential difference ΔE can thus serve as an indicator for quantitative analysis of the degree of nucleic acid hybridization.

### (Embodiment 2)

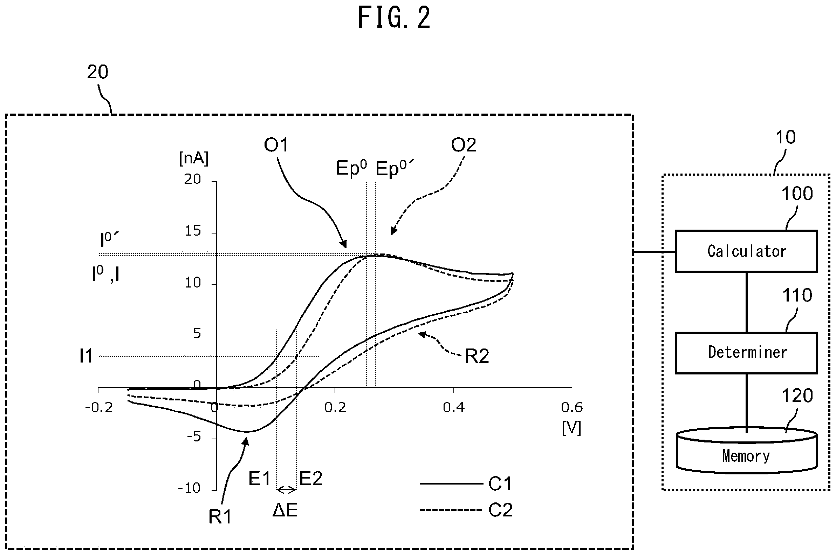

Fig. 2 is a diagram illustrating a device 10 according to embodiment 2. The device 10 of embodiment 2 is identical to the device 10 of embodiment 1, except for the following points. In the graph of the measurement system 20 of Fig. 2, the ordinate represents the current (nA) of the voltammogram and the abscissa represents the potential (V) of the voltammogram.

The device 10 includes a determiner 110. The determiner 110 determines the degree of nucleic acid hybridization based on the potential difference ΔE. As explained above regarding the reason for the potential difference ΔE, the degree of nucleic acid hybridization is greater with a larger potential difference ΔE and lower with a smaller potential difference ΔE. The determiner 110 therefore determines that the degree of nucleic acid hybridization is greater with a larger potential difference ΔE and the degree of nucleic acid hybridization is lower with a smaller potential difference ΔE.

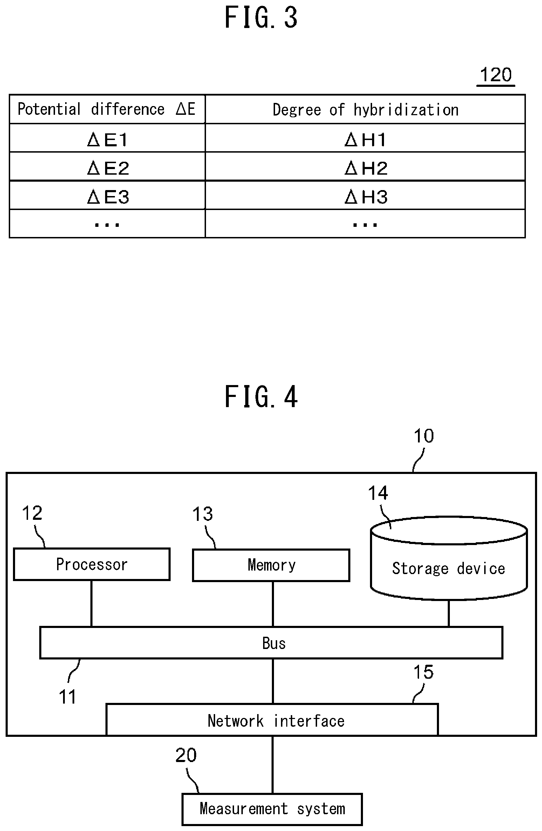

The device 10 also includes a memory 120. As explained below for Fig. 3, the determiner 110 may determine the degree of nucleic acid hybridization by comparing data representing the potential difference ΔE with reference data stored in the memory 120.

Fig. 3 is a diagram illustrating an example of reference data stored in the memory 120 shown in Fig. 2.

The reference data indicates degrees of nucleic acid hybridization (ΔH1, ΔH2, ΔH3, ... in the right column of Fig. 3) and their matching potential differences (ΔE1, ΔE2, ΔE3, ... in the left column of Fig. 3). The reference data can be generated beforehand by measurement using the measurement system 20 shown in Fig. 2. The determiner 110 shown in Fig. 2 may also compare the potential difference ΔE obtained from the measurement system 20 with the reference data shown in Fig. 3. The determiner 110 may determine the degree of nucleic acid hybridization based on this comparison.

Fig. 4 is a diagram showing the hardware configuration of a device 10. The device 10 includes a bus 11, processor 12, memory 13, storage device 14 and network interface 15.

The bus 11 is a data transmission line for sending and receiving of data between the processor 12, memory 13, storage device 14 and network interface 15. However, the method of connection between the processor 12, memory 13, storage device 14 and network interface 15 is not limited to being a bus connection.

The processor 12 is a computing device such as a CPU (Central Processing Unit) or GPU (Graphics Processing Unit). The memory 13 is a main memory unit such as a RAM (Random Access Memory) or ROM (Read Only Memory). The storage device 14 is an auxiliary storage device such as an HDD (Hard Disk Drive), SSD (Solid State Drive) or memory card.

The storage device 14 stores a program module that carries out the functions of the device 10 (for the calculator 100, determiner 110 or memory 120). The processor 12 executes each program module by reading from a memory 13, and carries out the function corresponding to each program module.

The network interface 15 is an interface for connection of the device 10 with a communication network such as a LAN (Local Area Network) or WAN (Wide Area Network). The device 10 is able to communicate with the measurement system 20 by connection to the communication network via the network interface 15. The device 10 may be connected to the measurement system 20 through a wireless network or it may be connected to the measurement system 20 through a wired network. According to another example, data obtained at the measurement system 20 (for example, the voltammogram C1 and the voltammogram C2 shown in Fig. 1) may be stored in a storage device (for example, a USB flash drive), and the device 10 may analyze the data stored in the storage device.

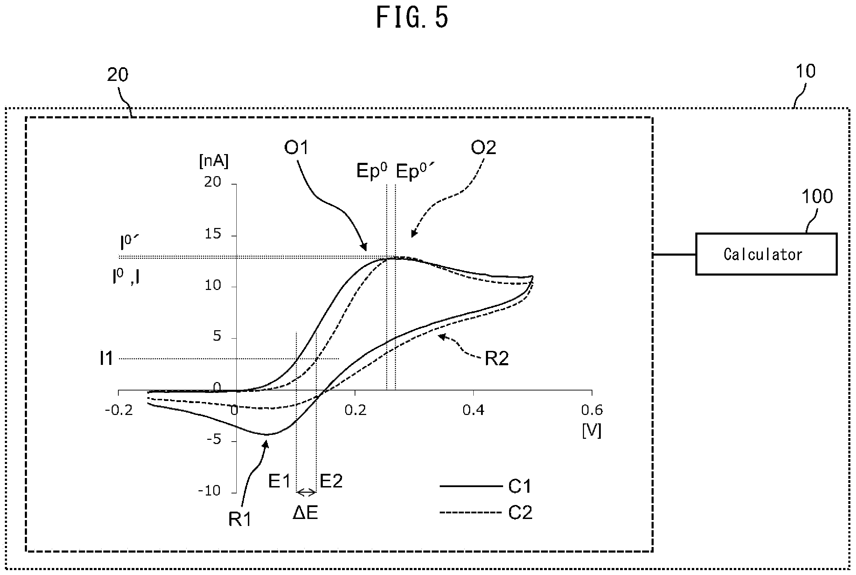

Fig. 5 is a diagram showing the first modified example of Fig. 1. In the graph of the measurement system 20 of Fig. 5, the ordinate represents the current (nA) of the voltammogram and the abscissa represents the potential (V) of the voltammogram.

As shown in Fig. 5, the device 10 may include a measurement system 20. In the example shown in Fig. 5, the device 10 may not only analyze the potential difference ΔE but may also measure the voltammogram C1 and the voltammogram C2.

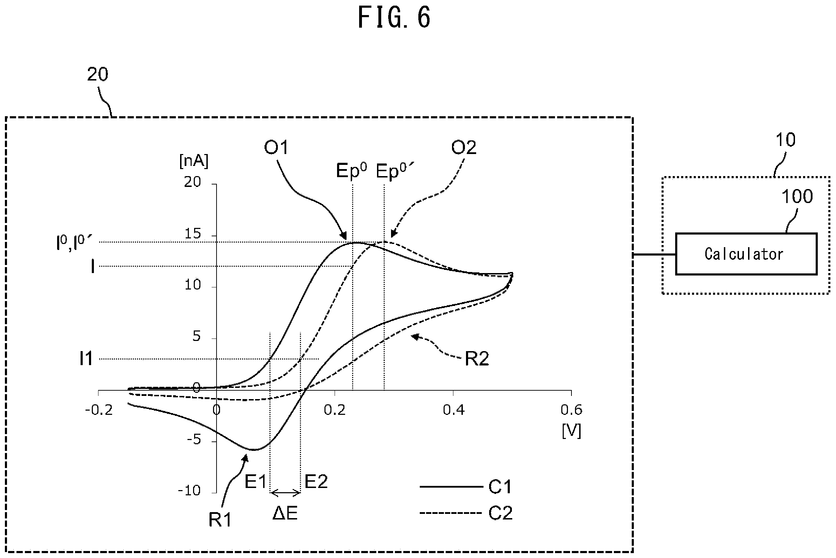

Fig. 6 is a diagram showing the second modified example of Fig. 1. In the graph of the measurement system 20 of Fig. 6, the ordinate represents the current (nA) of the voltammogram and the abscissa represents the potential (V) of the voltammogram.

The example shown in Fig. 6 differs from the example of Fig. 1 in terms of the graph in the measurement system 20. As shown in Fig. 6, even with a large difference between the peak current value I⁰ of the first oxidation wave O1 and the current value I of the second oxidation wave O2 (the second oxidation wave O2 has a current value I with potential Ep⁰ at the peak current value I⁰ of the first oxidation wave O1), the calculator 100 is able to calculate the potential difference ΔE in the same manner as the method described in relation to Fig. 1.

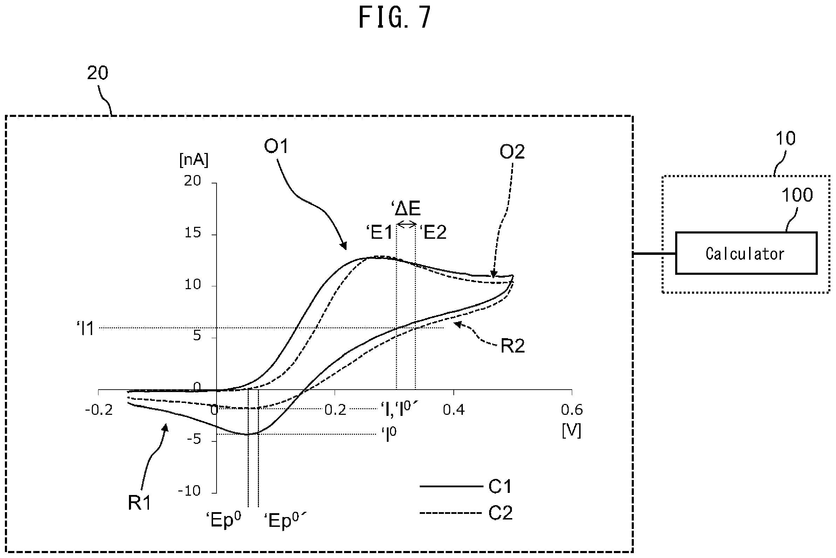

Fig. 7 is a diagram showing the third modified example of Fig. 1. In the graph of the measurement system 20 of Fig. 7, the ordinate represents the current (nA) of the voltammogram and the abscissa represents the potential (V) of the voltammogram.

As shown in Fig. 7, the device 10 can also detect the degree of nucleic acid hybridization using the first reduction wave R1 and the second reduction wave R2 instead of the first oxidation wave O1 and the second oxidation wave O2. The device 10 includes a calculator 100. The calculator 100 calculates the potential difference 'ΔE between the first potential 'E1 of the first reduction wave R1 and the second potential 'E2 of the second reduction wave R2. In the first reduction wave R1, the first potential 'E1 has a first current value 'I1 in a range of greater than the potential 'Ep⁰ at the peak current value 'I⁰ of the first reduction wave R1. In the second reduction wave R2, the second potential 'E2 has a first current value 'I1 in a range of greater than the potential 'Ep^{0'} at the peak current value 'I^{0'} of the second reduction wave R2.

The potential difference 'ΔE for the first reduction wave R1 and the second reduction wave R2 can be estimated as the degree of shift from the pre-hybridization reduction wave (the first reduction wave R1 in the example shown in Fig. 7) to the post-hybridization reduction wave (the second reduction wave R2 in the example shown in Fig. 7), similar to the potential difference ΔE for the first oxidation wave O1 and the second oxidation wave O2. Therefore, the potential difference 'ΔE for the first reduction wave R1 and the second reduction wave R2 can serve as an indicator for quantitative analysis of the degree of nucleic acid hybridization, similar to the potential difference ΔE for the first oxidation wave O1 and the second oxidation wave O2.

In the graph shown in Fig. 7, the second reduction wave R2 has a current value 'I for the potential 'Ep⁰ at the peak current value 'I⁰ of the first reduction wave R1.

### EXAMPLES

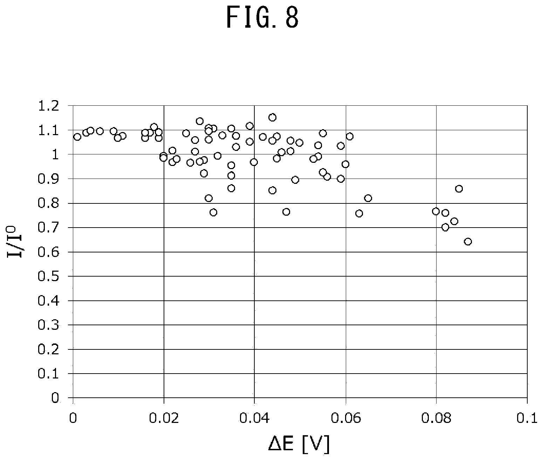

Fig. 8 is a diagram showing the correlation between miRNA hybridization analysis using the current value ratio I/I⁰ and miRNA hybridization analysis using the potential difference ΔE. In the graph of Fig. 8, the ordinate represents current value ratio I/I⁰ and the abscissa represents potential difference ΔE.

For each of 74 different measurement systems 20 (work electrodes) in the example shown in Fig. 8, the peak current ratio I/I⁰ and potential difference ΔE were calculated under the following conditions.
First oxidation wave O1: Oxidation wave before miRNA hybridization (measuring solution: 0.25 mM phosphate buffer + 0.5 mM NaClO₄, marker: 1 mM [Fe(CN)₆]⁴⁻)
Second oxidation wave O2: Oxidation wave after miRNA hybridization (measuring solution: 0.25 mM phosphate buffer + 0.5 mM NaClO₄, marker: 1 mM [Fe(CN)₆]⁴⁻)
CV sweep rate: 500 mV/sec
First current value I1: 3nA (approximately 20% of peak current value I⁰ for each first oxidation wave O1)

The median of the current value ratio I/I⁰ was 1.0205. This shows that the current value I of the second oxidation wave O2 was essentially equivalent to the peak current value I⁰ of the first oxidation wave O1. It can therefore be difficult to quantitatively analyze the degree of nucleic acid hybridization to a high degree of precision using the current value ratio I/I⁰.

The median of the potential difference ΔE was 35 mV. A potential difference ΔE on this level can be considered to be distinctly apparent. It is therefore concluded that the degree of nucleic acid hybridization can be quantitatively analyzed to high precision using the potential difference ΔE.

Embodiments of the invention have been described above with reference to the accompanying drawings, but it is to be understood that these are merely examples of the invention, and that other constructions may be employed as well.

The present application claims priority based on Japanese Patent Application No. 2018-183439, which was filed on September 28, 2018 and the entirety of the disclosure of which is incorporated herein by reference.

### [REFERENCE SIGNS LIST]

10 Device
11 Bus
12 Processor
13 Memory
14 Storage device
15 Network interface
20 Measurement system
100 Calculator
110 Determiner
120 Memory

## Claims

1. A method for detecting a degree of nucleic acid hybridization using a first oxidation wave and a second oxidation wave, wherein: the method includes calculating the potential difference between a first potential at which, in the first oxidation wave, a first current value is obtained in a range below the potential for the peak current value of the first oxidation wave, and a second potential at which, in the second oxidation wave, the first current value is obtained in a range below the potential for the peak current value of the second oxidation wave.

2. The method for detecting a degree of nucleic acid hybridization according to claim 1, which includes determining the degree of nucleic acid hybridization based on the potential difference.

3. The method for detecting a degree of nucleic acid hybridization according to claim 2, wherein determining the degree of nucleic acid hybridization includes comparing data representing the potential difference with reference data representing potential differences associated with degrees of nucleic acid hybridizations.

4. The method for detecting a degree of nucleic acid hybridization according to any one of claims 1 to 3, wherein:
the first oxidation wave represents the measurement results for a sample before hybridization, and
the second oxidation wave represents the measurement results for the sample after the hybridization.

5. The method for detecting a degree of nucleic acid hybridization according to any one of claims 1 to 4, wherein:
the second oxidation wave has a second current value for the potential at the peak current value of the first oxidation wave, and
the second current value of the second oxidation wave is 90% to 110% of the peak current value of the first oxidation wave.

6. The method for detecting a degree of nucleic acid hybridization according to any one of claims 1 to 5, wherein the nucleic acid is RNA or DNA.

7. The method for detecting a degree of nucleic acid hybridization according to claim 6, wherein the nucleic acid is miRNA.

8. A device for detecting a degree of nucleic acid hybridization using a first oxidation wave and a second oxidation wave, wherein:
the device comprises a calculator which calculates the potential difference between a first potential at which, in the first oxidation wave, a first current value is obtained in a range below the potential for the peak current value of the first oxidation wave, and a second potential at which, in the second oxidation wave, the first current value is obtained in a range below the potential for the peak current value of the second oxidation wave.

9. A program that causes a computer to function as a device for detecting a degree of nucleic acid hybridization using a first oxidation wave and a second oxidation wave, wherein:
the program causes the computer to:
calculate the potential difference between a first potential at which, in the first oxidation wave, a first current value is obtained in a range below the potential for the peak current value of the first oxidation wave, and a second potential at which, in the second oxidation wave, the first current value is obtained in a range below the potential for the peak current value of the second oxidation wave.
